(19) **Europäisches Patentamt / European Patent Office / Office européen des brevets**

(11) **EP 3 025 152 B1**

(12) **FASCICULE DE BREVET EUROPEEN**

(45) Date de publication et mention de la délivrance du brevet:
**07.06.2017 Bulletin 2017/23**

(21) Numéro de dépôt: **14744006.9**

(22) Date de dépôt: **04.07.2014**

(51) Int Cl.:
**G01N 29/06** (2006.01)   **A61B 8/08** (2006.01)
**G01S 15/89** (2006.01)

(86) Numéro de dépôt international:
**PCT/EP2014/064346**

(87) Numéro de publication internationale:
**WO 2015/010878 (29.01.2015 Gazette 2015/04)**

(54) **PROCÉDÉ ET DISPOSITIF ULTRASONORES POUR REPRÉSENTER LA PROPAGATION D'ONDES ULTRASONORES DANS UN GUIDE D'ÉPAISSEUR LINÉAIREMENT VARIABLE**

VERFAHREN UND VORRICHTUNG ZUR DARSTELLUNG DER AUSBREITUNG VON ULTRASCHALLWELLEN IN EINEM WELLENLEITER MIT LINEAR VARIIERENDER DICKE

ULTRASONIC METHOD AND APPARATUS FOR REPRESENTAION OF ULTRASOUND WAVE PROPAGATION IN A GUIDE WITH LINEARLY VARYING THICKNESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2013 FR 1357204**

(43) Date de publication de la demande:
**01.06.2016 Bulletin 2016/22**

(73) Titulaires:
• **Azalee**
  **75005 Paris (FR)**
• **Centre National de la Recherche Scientifique (C.N.R.S.)**
  **75794 Paris Cedex 16 (FR)**
• **Université Pierre et Marie Curie (Paris 6)**
  **75252 Paris Cedex 05 (FR)**

(72) Inventeurs:
• **MOREAU, Ludovic**
  **38100 Grenoble (FR)**
• **MINONZIO, Jean-Gabriel**
  **F-75014 Paris (FR)**
• **TALMANT, Maryline**
  **F-27200 Vernon (FR)**
• **LAUGIER, Pascal**
  **F-75017 Paris (FR)**

(74) Mandataire: **Osha Liang**
  **2, rue de la Paix**
  **75002 Paris (FR)**

(56) Documents cités:
  **WO-A1-03/099133**

• **JOSQUIN FOIRET ET AL: "Guided mode measurement on bone phantoms with realistic geometry", ULTRASONICS SYMPOSIUM (IUS), 2011 IEEE INTERNATIONAL, IEEE, 18 octobre 2011 (2011-10-18), pages 1610-1613, XP032230494, DOI: 10.1109/ULTSYM.2011.0400 ISBN: 978-1-4577-1253-1**
• **JIANGANG CHEN ET AL: "Paper;Measurement of guided mode wavenumbers in soft tissue bone mimicking phantoms using ultrasonic axial transmission;Measurement of guided mode wavenumbers in soft tissue--bone mimicking phantoms using ultrasonic axial transmission", PHYSICS IN MEDICINE AND BIOLOGY, INSTITUTE OF PHYSICS PUBLISHING, BRISTOL GB, vol. 57, no. 10, 26 avril 2012 (2012-04-26), pages 3025-3037, XP020221934, ISSN: 0031-9155, DOI: 10.1088/0031-9155/57/10/3025**

**Description**

L'invention concerne, en général, la représentation dans un repère fréquence-nombre d'onde, de la propagation d'une ou plusieurs ondes ultrasonores dans un guide, ou modes guidés, dans le cas où l'épaisseur du guide est linéairement variable.

L'invention concerne, en particulier, la représentation d'un ou plusieurs modes guidés par le cortex ou partie corticale d'un os long humain, dans la mesure où un tel os peut être approximé par un guide d'épaisseur linéairement variable. L'invention vise notamment à aider à l'évaluation du risque de fracture, de façon non intrusive dans le corps humain, de l'ostéoporose, en estimant les propriétés élastiques et géométriques (notamment épaisseur) de l'os, à partir de la représentation des modes guidés, en nombre d'onde ou en vitesse de phase, en fonction de la fréquence temporelle.

Dans la demande, « polynôme de degré n de la variable x » désignera une fonction mathématique $f(x)= C_0 + C_1.x + ... + C_n.x^n$ où $C_0$ est le coefficient de degré 0 en x du polynôme, où $C_1$ est le coefficient de degré 1 du polynôme qui pourra, dans la demande, être noté C sans indice et où $C_n$ est le coefficient de degré n du polynôme.

Dans la demande «guide en dièdre» désignera un guide pour les ondes ultrasonores, d'épaisseur linéairement variable, notamment un guide limité par deux plans en coin, présentant un défaut de parallélisme entre les plans, défaut limité à quelques degrés d'angle.

Dans la demande, norme d'un vecteur à « n » composantes dans un espace à « n » dimensions, désigne la norme euclidienne ou norme 2 de ce vecteur, égale à la racine carrée de la somme des carrés des composantes du vecteur.

Dans la demande le mot « Pi » ou le symbole « $\pi$» désignera le rapport du périmètre d'un cercle à son diamètre.

Dans la demande « nombre d'onde », désignera le nombre de périodes spatiales ou longueur d'onde d'une onde sur le périmètre d'un cercle de diamètre 1, noté k avec $k= \frac{2.\pi}{\lambda}$ où $\lambda$ est la longueur d'onde.

Dans la demande « pulsation d'une onde » désignera le nombre de périodes temporelles d'une onde sur le périmètre d'un cercle de diamètre 1, notée $\omega$ avec $\omega=2.\pi.f$ où f est la fréquence temporelle, inverse de la période temporelle T, avec f= 1/T.

Dans la demande « vitesse de phase » désignera le rapport de la pulsation d'une onde ultrasonore à son nombre d'onde, notée $v_\varphi = \frac{\omega}{k}$.

Une onde plane sinusoïdale et progressive dans la direction des x croissants sera définie sur M récepteurs positionnés à des valeurs discrètes de la variable spatiale x, par son amplitude complexe proportionnelle à: $W(t,x) = e^{-i(\omega.t-k.x)}$.

Dans la demande, « Transformée de Fourier temporelle discrète pour la pulsation $\omega$ » désignera, pour un signal V(t) défini sur des valeurs discrètes de la variable temporelle t, le produit scalaire du vecteur des valeurs de V(t) et du vecteur des valeurs d'une onde sinusoïdale $T(t) = e^{+i.\omega.t}$ sur les valeurs discrètes du temps, avec i racine complexe de -1 et $\omega$ pulsation de l'onde.

Dans la demande, « Transformée de Fourier spatiale discrète dans la direction spatiale x pour le nombre d'onde k réel, constant » désignera, pour un signal V(x) défini sur M récepteurs positionnés à des valeurs discrètes de la variable spatiale x, le produit scalaire du vecteur des valeurs de V(x) et du vecteur des valeurs d'une onde plane $T(x) = \frac{1}{\sqrt{M}}.e^{-ikx}$ sur les récepteurs, avec i racine complexe de -1 et k nombre d'onde de l'onde plane. Le vecteur T(x) est de norme euclidienne égale à 1.

Dans la demande, « Transformée de Fourier spatiale discrète modifiée, dans la direction spatiale x pour le nombre d'onde k(x) réel, variable selon x» désignera , pour un signal V(x) défini sur M récepteurs positionnés à des valeurs discrètes de la variable spatiale x, le produit scalaire du vecteur des valeurs de V(x) et du vecteur des valeurs d'une onde plane modifiée $T(x) = \frac{1}{\sqrt{M}}.e^{-ik(x).x}$ sur les récepteurs, avec i racine complexe de -1 et k(x) nombre d'onde variable avec x. Le vecteur T(x) est de norme euclidienne égale à 1.

Dans la demande, on désignera, pour une matrice rectangulaire S à M lignes et N colonnes, la matrice pouvant être carrée si M=N, par « Décomposition en valeurs singulières de rang numérique R », avec R inférieur ou égal au minimum de M et de N, une décomposition en valeurs singulières de la matrice S, réduite numériquement à R valeurs singulières, les plus grandes, soit en précisant le nombre R des valeurs singulières à conserver en partant de la plus élevée pour des valeurs singulières classées par ordre décroissant, soit en précisant un seuil, choisi en considération du bruit, en-dessous duquel une valeur singulière, est négligée. La décomposition en valeurs singulières produit notamment des vecteurs singuliers de sortie ou de réception, associés aux valeurs singulières, qui sont orthogonaux entre eux et normés chacun à l'unité et qui seront interprétés dans la suite comme une base de l'espace des signaux reçus ou enregistrés ou de sortie. La décomposition en valeurs singulières produit aussi des vecteurs singuliers d'émission ou d'entrée, associés aux valeurs singulières, qui sont orthogonaux entre eux et normés chacun à l'unité et qui seront interprétés dans la suite comme une base de l'espace des signaux émis ou d'entrée.

Dans la demande, on désignera par capteur ultrasonore ou émetteur ultrasonore ou récepteur ultrasonore, respectivement un capteur, émetteur ou récepteur à effet piézo-électrique ou, de façon équivalente, utilisant tout autre effet apte à capter émettre ou recevoir une onde ultrasonore et la convertir en signal électrique puis numérique.

L'art antérieur à l'invention connaît notamment la demande de brevet français publiée sous le N° FR2946753, un dispositif et un procédé de représentation des modes de propagation d'une onde ultrasonore dans un guide plan et parallèle, d'épaisseur constante, dans l'optique d'utiliser cette représentation pour mesurer des caractéristiques physiques d'un os long humain et notamment de sa couche corticale.

Cet art antérieur utilise une décomposition en valeurs singulières des transformées de Fourier temporelles d'un signal ultrasonore sur des récepteurs, en provenance d'émetteurs, et une projection d'une onde plane de vecteur d'onde constant dans la base des vecteurs singuliers de réception , pour améliorer la détermination de la propagation de l'onde ultrasonore dans un guide plan et à faces parallèles, d'épaisseur constante, comme une plaque et l'appliquer à un os.

L'application, à un os long, de cette méthode antérieure, qui est uniquement adaptée à un guide plan et parallèle, d'épaisseur constante, induit toutefois des biais ou erreurs pour l'art antérieur.

En effet, un os long humain a une épaisseur corticale variable qui impose de le considérer, pour une onde ultrasonore reçue par une barrette ou sonde linéaire de capteurs ultrasonores, grossièrement alignée avec la direction la plus étendue de l'os long, comme un dièdre de faible angle au sommet (0° à 3° d'angle, avec une valeur courante de 1° d'angle). Dans un tel guide, l'onde ultrasonore se propageant par convention vers le sommet du dièdre n'est pas, en pratique, assimilable à une onde plane sauf sur une partie infinitésimale du guide. Il en résulte une erreur systématique ou biais sur la détermination de la représentation des modes de propagation d'un os long avec la méthode de l'art antérieur, ceux-ci étant notamment différents des modes d'une plaque d'épaisseur constante.

Le traitement de signal de l'art antérieur ci-dessus se révèle ainsi insuffisant pour mesurer avec précision les modes de propagation d'un os humain, en tant que guide d'épaisseur linéairement variable.

L'art antérieur connaît aussi des tentatives pour approximer localement un guide en dièdre à un guide plan parallèle, en limitant le nombre de récepteurs utilisés pour représenter les ondes guidées, par des sous-fenêtres. Toutefois, cette stratégie est fortement pénalisante en matière de rapport signal/bruit et de résolution, qui se dégradent lorsque le nombre de récepteurs et donc de signaux diminue. L'os est de plus, un milieu absorbant et de géométrie irrégulière, ce qui limite le nombre possible de récepteurs. Ces tentatives sont ainsi affectées, en pratique, de nombreux inconvénients, principalement dus à des distances entre émetteurs et récepteurs nécessairement trop importantes et une nécessité de connaître l'épaisseur du guide sous chaque sous-fenêtre de réception.

Il n'existe donc pas dans l'art antérieur de dispositif ou de procédé pour représenter les modes de de propagation d'un guide en dièdre, qui serait pourtant directement applicable à la mesure ultrasonore des modes guidés par un os long humain.

Avantageusement, une telle méthode de représentation des modes de propagation d'un guide en dièdre devrait être obtenue à partir de capteurs ultrasonores piézo-électriques, s'étendant en une barrette linéaire dans une direction, sur une droite et selon un pas régulier, pour tirer parti d'éléments existant dans l'art antérieur.

Dans ce contexte, l'invention concerne :

Un dispositif pour la représentation, dans un repère fréquence-nombre d'onde f-k, de la propagation d'une onde ultrasonore dans un guide en dièdre, qui comprend des émetteurs ultrasonores référencés par « Ej » avec j entier variant entre 1 et N, N entier strictement positif et des récepteurs ultrasonores référencés par « Ri » avec i entier variant entre 1 et M, M entier strictement positif, les récepteurs étant disposés spatialement sur un premier segment d'une droite selon un pas régulier « A », qui comprend des moyens de traitement du signal reçu par les récepteurs, en provenance des émetteurs, dans lequel les moyens de traitement comprennent des moyens de calcul d'une transformée de Fourier spatiale discrète modifiée, pour une variable spatiale d'intégration « x », centrée au milieu dudit premier segment et parcourant les récepteurs dans le sens des x croissants, et pour un vecteur d'onde $k(x)$ égal à un produit $k.P(x)$, avec k coefficient constant en x et compris entre 0 et $2*Pi/A$, et avec $P(x)$ polynôme en x, de coefficient de degré 0 en x égal à 1 et de coefficient « C » de degré 1 en x tel que C.A est compris entre -1/10 et +1/10.

Dans des variantes du dispositif ci-dessus :

- les émetteurs sont disposés sur ladite droite.

- les émetteurs sont disposés sur ladite droite selon le pas «A» et forment avec les récepteurs une barrette s'étendant linéairement dans la direction de ladite droite.

- les émetteurs et les récepteurs sont des capteurs piézo-électriques.

- les moyens de traitement du signal comprennent des convertisseurs d'un signal analogique en signal numérique

- les moyens de calcul sont numériques

L'invention concerne aussi un procédé d'utilisation du dispositif ci-dessus pour la représentation, dans un repère fréquence-nombre d'onde f-k, de la propagation d'une onde ultrasonore dans un guide en dièdre, à une fréquence temporelle f0 et à un nombre d'onde k0, comprenant les étapes suivantes :

- émettre dans le guide, un signal ultrasonore fonction du temps et à large bande passante incluant la fréquence « f0 », par un émetteur Ej avec j entier choisi entre 1 et N;

- recevoir, le signal propagé dans le guide, en fonction du temps, sur les récepteurs Ri avec i entier choisi entre 1 et M;

- calculer une transformée de Fourier temporelle discrète du signal reçu à la fréquence f0 et ranger le résultat Sij dans la i-ième ligne et la j-ième colonne d'une matrice rectangulaire S(f0) à M lignes et N colonnes;

- remplir la matrice S(f0), en répétant les opérations précédentes pour les récepteurs autres que Ri et pour les émetteurs autres que Ej;

- décomposer la matrice S(f0) en valeurs singulières en fixant un rang numérique « R » de la matrice S, avec R entier choisi entre 1 et N, pour obtenir un nombre R de vecteurs singuliers de réception référencés par « Ur (f0)» avec r entier variant entre 1 et R ;

- calculer une transformée de Fourier spatiale discrète modifiée des vecteurs singuliers de réception Ur (f0), pour une variable spatiale d'intégration « x » centrée au milieu dudit premier segment et parcourant les récepteurs et pour un vecteur d'onde k(x) égal à k0, choisi entre 0 et 2.Pi/A, multiplié par un polynôme en x « P(x) », de coefficient de degré 0 égal à 1 et de coefficient « C » de degré 1 en x tel que C.A est variable entre -1/10 et +1/10 ;

- calculer une fonction « Norme(f0,k0,C) », égale à la somme des modules carrés des transformées de Fourier spatiales discrètes modifiées des vecteurs singuliers de réception Ur (f0) sur la plage de variation de C,

- déterminer la valeur C=Cmax pour laquelle Norme(f0,k0,C) est maximum, sur la plage de variation de C;

- représenter dans le repère f-k, la propagation de l'onde ultrasonore dans le guide en dièdre, en f0 et k0, en reportant la valeur du maximum Norme(f0,k0,Cmax) au point (f0,k0) du repère.

L'invention concerne aussi une application du procédé ci-dessus pour obtenir la représentation, dans le repère f-k, de la propagation des ondes ultrasonores dans le guide en dièdre, en reportant la valeur du maximum Norme(f,k,Cmax(f,k)) au point (f,k) du repère, pour k variant entre 0 et 2.Pi/A et f variant sur les fréquences de la large bande passante.

L'invention concerne aussi une application du procédé ci-dessus dans laquelle le guide en dièdre est un os long humain.

L'invention est décrite en liaison avec des figures numérotées 1 à 3:

La figure 1 qui représente un guide en dièdre (1) surmonté d'une barrette linéaire de capteurs en contact avec le guide, divisée en un ensemble d'émetteurs (2) et un ensemble de récepteurs (3).

La figure 2 montre la représentation des modes de propagation d'un guide avec un angle de dièdre de 2° pour l'art antérieur, notamment la demande de brevet FR2946753 et pour une épaisseur au milieu des récepteurs égale à 2,2mm. Le matériau du guide est un fantôme d'os commercial, composé de fibres de verre dans un époxy.

La figure 3 montre la représentation des modes de propagation du même guide que la figure 2 avec l'invention.

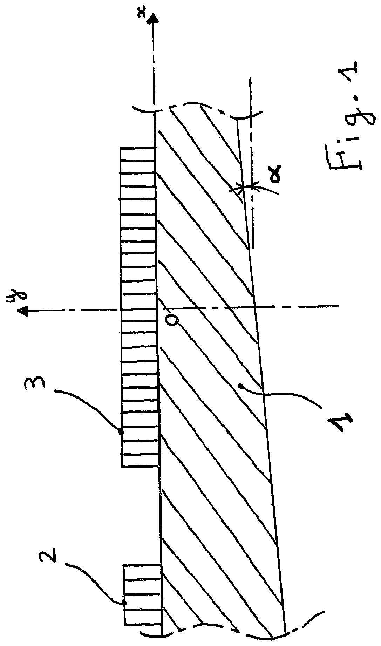

En référence à la figure 1, dans un repère Oxyz orthonormé d'un espace euclidien, un guide en dièdre(1) est composé d'un matériau comme de l'os humain ou un matériau fantôme d'un tel os comme des fibres de verre enrobées d'un matériau époxy, disponible dans le commerce. Le guide en dièdre s'étend entre un premier plan xOz et un second plan passant par le point x=0, y=-e0, z=0, parallèle à l'axe Oz et faisant, en projection dans le plan xOy, un angle « $\alpha$ » ou alpha = +2° d'angle avec l'axe Ox, dans le plan xOy orienté pour les angles dans le sens trigonométrique direct. Le guide est ainsi d'une épaisseur e fonction de x, donnée par la formule : e(x)= e0.(1- tan(alpha).x), e diminuant vers les x croissants. Pour cet exemple, eO vaut 2,2 mm.

Un jeu d'émetteurs (2) en nombre N égal à 5 est disposé régulièrement selon un pas « A » de 0,8mm sur l'axe des x à des valeurs négatives de x et sont séparés par une zone absorbante pour les ondes acoustiques, d'un ensemble de récepteurs (3) en nombre M égal à 24, régulièrement espacés selon le pas A, entre les positions x=-11,5.A et x=11,5.A, par pas de A, de façon symétrique autour de l'origine O du repère, sur l'axe des x. Le pas A fixe la valeur maximale du nombre d'onde k=2.Pi/A= 7,8 mm-1 que les récepteurs peuvent détecter.

Les émetteurs sont reliés de façon connue à un générateur de signal à travers un multiplexeur permettant de sélectionner séquentiellement dans le temps un des émetteurs parmi les N.

Les récepteurs sont reliés de façon connue à un convertisseur analogique-numérique à travers un démultiplexeur, électronique multi-voies, permettant de numériser séquentiellement dans le temps, le signal des récepteurs, sous forme de données numériques.

Le convertisseur analogique-numérique est complété dans les moyens de traitement par un calculateur et des moyens de calcul, aptes à traiter numériquement par programme les données selon des méthodes mathématiques et par des moyens d'affichage sur écran des données, en fausses couleurs ou en niveaux de gris ou en courbes de niveaux ou dans une représentation à trois axes.

Les moyens de traitement sont aussi capables d'activer un émetteur pour faire propager un signal ultrasonore, large bande et incluant une fréquence temporelle « f0 » donnée, dans le guide en dièdre vers les épaisseurs décroissantes, de numériser dans un vecteur à M composantes, le signal reçu par chaque récepteur en fonction du temps, en provenance de l'émetteur et d'en calculer la transformée de Fourier temporelle discrète à la fréquence f0.

Les moyens de traitement sont aussi capables de façon connue d'activer successivement chaque émetteur et numériser les signaux reçus sur les récepteurs pour former un ensemble de N vecteurs de signaux de réception à M composantes qui rassemblent dans un tableau à M lignes et N colonnes, les transformées de Fourier temporelle à f0, des signaux reçus par les M récepteurs en provenance des N émetteurs, et de les stocker dans un tableau adressable par les moyens de calcul sous le nom de « matrice S(f0) » ou « S(f0)».

Les moyens de traitement peuvent aussi être capables de calculer la transformée de Fourier du signal sur un ensemble de fréquences de la bande large du signal émis et de stocker ces valeurs en vue de calculer un ensemble de matrices S(f) référencées par un ensemble de fréquences f choisies dans la bande du signal émis. Cette stratégie permet de procéder en parallèle pour toutes les fréquences d'intérêt et d'obtenir un tenseur à trois dimensions S(f). Cette stratégie permet d'éviter de réémettre pour chaque fréquence d'intérêt et de tirer parti au mieux du signal large bande d'émission.

L'ensemble des opérations matérielles aboutissant à rendre disponibles dans un tableau et une matrice S(f0), les transformées de Fourier temporelles à f0, des signaux des récepteurs ayant traversé le guide en dièdre après avoir été émis par un émetteur, est désigné ci-dessous par « tir ».

A l'issue d'un tir, les moyens de calculs disposent à f0, d'un tableau ou matrice S(f0) rectangulaire de dimensions MxN avec M supérieur ou égal à N, dans le mode décrit.

De façon connue de l'art antérieur, il est possible de calculer la décomposition de la matrice S(f0) en valeurs singulières, réduite à un rang numérique R inférieur ou égal à N. Cette opération est connue dans de nombreuses bibliothèques mathématiques et le choix empirique du rang R peut être fait en fonction des conditions de bruit rencontrées par l'homme du métier, par de simples essais de routine.

Dans cette opération R valeurs singulières sont calculées et R vecteurs singuliers de réception à M composantes sont calculés, ces vecteurs sont orthogonaux entre eux et normés à 1.

On projette alors, sur les vecteurs singuliers de réception, une série de vecteurs tests correspondant à des ondes planes modifiées dont le nombre d'onde est un vecteur k(x) égal à un coefficient k0 constant en x qui est multiplié par un polynôme de degré 1 en x, dont le coefficient de degré 0 en x vaut 1 et dont le coefficient C de degré 1 en x est variable de façon que le produit C.A, de C et du pas A entre les récepteurs, varie entre -0,1 et +0,1, avec x centré au milieu du segment des récepteurs.

Cette opération est équivalente à former la transformée de Fourier spatiale discrète modifiée des vecteurs singuliers de réception.

D'une façon générale, la variation de C proposée pour l'invention vise à décrire au mieux, les fluctuations selon x du nombre d'onde, dans une zone de variation des modes d'un guide en dièdre appelée plage de « variation adiabatique des modes » pour des angles de dièdre correspondant à la variation linéaire d'épaisseur d'un os long. Dans cette approximation les ondes se propageant dans la direction x sont des ondes planes dans la direction x, dont le nombre d'onde est continument variable selon x. Toute approximation polynômiale ou fonctionnelle permettant de rendre compte d'une variation continue du nombre d'onde ou du nombre d'onde des modes d'un guide en dièdre ou d'un os long, selon une loi attendue, serait ainsi conforme à l'enseignement de l'invention.

Il resterait dans l'enseignement de l'invention d'augmenter le degré du polynôme au-delà de 1 pour améliorer la méthode par l'utilisation d'un vecteur $k(x) = k0.(1 + C.x + ... + C_n.x^n)$ où $k$ et $C, ..., C_n$ sont constants en x, non-nuls et inconnus à priori, mais au détriment du temps de calcul, comme expliqué ci-dessous. Pour une puissance de calcul supposée limitée et un fonctionnement pratique de présentation en temps réel des modes de propagation à l'écran dans l'approximation adiabatique, on ne considérera donc pas les termes de degré supérieurs à 1 dans P(x), dans l'exposé ci-dessous.

Pour des guides en dièdres d'angle faible représentatif de la variation de l'épaisseur corticale d'un os long, la requérante a ainsi calculé que les modes d'un tel guide ont un nombre d'onde qui varie en fonction de x avec un nombre d'onde $k(x)$ qui peut être approximé utilement par $k(x) = k0.(1 + C.x + ... + C_n.x^n)$ où $k$ et $C, ..., C_n$ sont constants en x et inconnus à priori et où k0 est le nombre d'onde en x=0.

Les coefficients du polynôme sont néanmoins fonctions de la fréquence temporelle et du mode guidé considéré, ce qui ne permet pas une approximation a priori, par un polynôme uniquement fonction de l'angle du dièdre, dans un repère f-k.

Cette approximation peut être ramenée à un polynôme $k(x) = k0.(1 + C.x)$ pour l'essentiel des cas pratiques avec k0 compris entre 0 et 2.Pi/A où A est le pas des récepteurs et C est variable de façon que C.A reste compris entre -0.1 et +0.1 sur toute la plage de variation adiabatique des modes de propagation et majoritairement entre -0.05 et +0,05 pour la plupart des cas rencontrés pour l'os humain.

Dans le cas particulier d'une propagation vers les épaisseurs décroissantes, les valeurs de C sont majoritairement négatives, ce qui permet de restreindre le domaine de recherches des valeurs de C et diminuer le temps de calcul avec l'invention. De plus, la méthode selon l'invention est particulièrement efficace dans ce se sens de propagation.

La méthode de l'invention pour calculer les modes de propagation d'un guide en dièdre consiste alors essentiellement:

- à choisir dans le plan (f,k), un point (f0,k0) où l'on souhaite représenter les modes de propagation, avec f0 dans la bande passante de fréquence d'un signal d'émission à large bande et k0 constant en x et choisi entre 0 et 2.Pi/A où A est l'espacement entre des récepteurs régulièrement répartis dans une direction x

- à calculer la matrice S(f0)

- à la décomposer en valeurs singulières de rang numérique donné

- à calculer une transformée de Fourier spatiale discrète modifiée, des vecteurs singuliers de réception pour une variable spatiale d'intégration « x » centrée au milieu dudit premier segment et parcourant les récepteurs et pour un nombre d'onde variable en x k(x) égal au coefficient k0, constant en x et choisi entre 0 et 2.Pi/A, multiplié par un polynôme P(x) de coefficient de degré 0 égal à 1 et de coefficient « C » de degré 1 en x, variable de façon que C.A soit compris entre -1/10 et +1/10.

- à déterminer la valeur C=Cmax pour laquelle une fonction « Norme(f0,k0,C) », comprise entre 0 et 1, égale à la somme des modules carrés des transformées de Fourier spatiales discrètes modifiées des vecteurs singuliers de réception atteint un maximum de valeur Norme(f0,k0,Cmax) sur la plage de variation de C. Le cas échéant, le polynôme P(x) pourra être choisi avantageusement de degré supérieur à 1, si Cmax.A atteint son minimum (-10%) ou son maximum (+10%) pour k0,f0, au détriment du temps de calcul.

- à représenter les modes de propagation du guide d'épaisseur variable, dans un repère à deux dimensions f-k, au point (f0,k0) en reportant graphiquement la valeur du maximum Norme(f0,k0,Cmax) au point (f0,k0) dans f-k par une indication de niveau de gris ou d'une échelle de couleurs, le cas échéant une représentation à trois axes ou par lignes de niveau peut être utilisée.

La méthode ayant été détaillée pour obtenir une représentation en un point (k0,f0) du repère f-k, il est alors aisé de la répéter par l'opération consistant à :

- faire varier (f0, k0) pour parcourir le plan f-k et y représenter le signal en tout point souhaité (f,k), en reportant la valeur du maximum de Norme(f,k,Cmax(f,k)), dans les limites de variations fixées par A pour k et par la bande passante du signal d'émission pour f.

Il est à noter que la méthode permet de déterminer la présence des modes guidés dans le repère k-f sans a priori sur l'épaisseur et le matériau. Notamment on pourra déterminer, sans a priori, la présence de plusieurs modes à une même fréquence temporelle.

La représentation des modes peut, par exemple, être faite en associant un niveau de gris ou une intensité de couleur à des plages de valeurs entre 0 et 1 de la valeur de Norme(f,k,Cmax(f,k)) et en la présentant à l'écran à un opérateur, le cas échéant une représentation à trois axes ou par lignes de niveau peut être utilisée.

La représentation des modes est indépendante de l'opérateur, hormis le choix d'un rang numérique R fixé en fonction du rapport signal à bruit rencontré en pratique et le choix d'un seuil pour Norme(f,k,Cmax(f,k)), au-dessus duquel les valeurs de cette Norme sont conservées.

De façon équivalente, les moyens de traitement pourront, être complétés par un moyen pour un opérateur de choisir un seuil de représentation correspondant à une valeur de Norme(f,k,Cmax(f,k)) en dessous de laquelle, les modes ne sont pas représentés dans le plan f-k.

La méthode ou procédé de l'invention nécessite seulement de remplacer dans les calculs de k0, connues de l'art antérieur, par application de la décomposition en valeurs singulières, le vecteur d'onde ou nombre d'onde constant k0 des vecteurs de test par un vecteur d'onde variable en x, approximé par un polynôme de degré 1 multipliant k0 pour des valeurs de x faibles, ce qui correspond à un développement limité de k(x) d'ordre 1 en x autour de la valeur de k0 en x=0, au milieu du segment des récepteurs. Ensuite, on recherche un maximum absolu d'une fonction d'une variable C ou de plusieurs variables C, C2, ..., Cn au lieu d'une seule variable si un polynôme de degré supérieur à 1 est utilisé, correspondant à un développement limité d'ordre n de k(x) autour de k0. La norme choisie restant la même, la généralisation à plusieurs coefficients C,C2,...,Cn est donc particulièrement aisée. La méthode de l'invention est ainsi adaptable à la recherche de coefficients polynômiaux de degrés supérieurs à 1, si la puissance de calcul permet la recherche d'un maximum d'une fonction de plus d'une variable.

L'utilisation de la méthode de l'invention ne nécessite donc qu'un dispositif ultrasonore comprenant un moyen de calcul permettant de remplacer dans la transformée de Fourier spatiale en x pour un vecteur d'onde k0, le vecteur d'onde k0 par un vecteur d'onde k(x) avec k(x)=k0.(1+C.x) où C est variable de façon que le produit C.A, de C par le pas A entre les récepteurs, varie entre -10% et +10%.

A titre de cas particulier, on peut noter que la transformée de Fourier spatio-temporelle peut se retrouver en fixant N (nombre des émetteurs) à 1 et C à 0.

Dans le cas de la figure 1, le calcul pourra finalement être effectué de la façon ci-dessous, décrit par comparaison avec l'art antérieur.

Un signal à large bande passante contenant une fréquence f0 est appliqué à chaque émetteur ou transmetteur et le signal $s\left(t, x_i^R, x_j^E\right)$ correspondant est enregistré en fonction du temps sur chaque récepteur et il en est effectué la transformée de Fourier temporelle à la fréquence f0 des signaux de réception pour obtenir un signal Sij= $S\left(f0, x_i^R, x_j^E\right)$.

Après cette opération, il est obtenu une matrice S(f0) de N lignes, chaque ligne correspondant à un transmetteur donné et de M colonnes, chaque colonne correspondant à un récepteur donné, dont les valeurs S(f0,i,j) sont égales à la transformée de Fourier à la fréquence f0 du signal émis entre l' émetteur j et le récepteur i.

On procède ensuite à une décomposition en valeurs singulières de la matrice S(f0) et, par exemple, à un seuillage pour ne conserver qu'un nombre de valeurs singulières inférieur au nombre total de ces valeurs en éliminant des valeurs singulières les plus faibles inférieures à un seuil déterminé expérimentalement en fonction du bruit. Le rang de la matrice des valeurs singulières est alors fixé numériquement.

La décomposition en valeurs singulières permet d'obtenir en même temps que les valeurs singulières une base de vecteurs singuliers de réception et la suppression de certaines valeurs singulières permet ainsi d'obtenir un ensemble de vecteurs singuliers de réception orthogonaux et associés aux valeurs singulières conservées en nombre égal au rang, désigné par «R», de la matrice des vecteurs singuliers de réception.

Dans l'art antérieur, on sélectionne un vecteur test de nombre d'onde k0, dans la base des vecteurs singuliers de réception dont la norme est égale à 1. Ce vecteur test est de forme sur les récepteurs:

$$e_{test} = \frac{1}{\sqrt{M}} \exp\left(-i.k0.x\right)$$

Cette forme, correspond à une onde plane progressive sur les récepteurs, dans le sens des x positifs et de vecteur d'onde k0 constant.

La projection de ce vecteur test dans le sous-espace du signal, c'est-à-dire sur les vecteurs singuliers de réception permet alors d'obtenir une fonction qui est une norme fonction de f0 et de k0, qui s'exprime par:

$$Norme(f0, k0) = \sum_{n=1}^{R} |\langle e_{test}(k0)|U_n(f0)\rangle|^2$$

Cette opération de sommation est effectuée sur les vecteurs singuliers de réception et permet d'obtenir une Norme(f0,k0) pour le sens de propagation sur la zone de réception (vers les x croissants).

Elle correspond à une sommation des modules carrés des transformées de Fourier en k0, des vecteurs singuliers de réception. Elle correspond aussi à la norme euclidienne du vecteur test dans la base des vecteurs singuliers de réception.

La transformée de Fourier peut être ici interprétée comme un produit scalaire (noté « | ») du vecteur test et de chaque vecteur singulier. En pratique, la projection du vecteur test sur la base des vecteurs singuliers de réception est ainsi équivalente à effectuer la transformée de Fourier spatiale discrète des vecteurs singuliers.

Cette norme correspond donc aussi à la somme des carrés des normes des transformées de Fourier des vecteurs singuliers de réception.

Pour mettre en oeuvre l'invention, il suffit dans la méthode de l'art antérieur, de modifier le vecteur test choisi dans le calcul de la Norme par la nouvelle formule :

$$e_{test} = \frac{1}{\sqrt{M}} \exp(-i.k(x).x)$$

Avec k(x) = k0.(1+C.x) k0 choisi sur entre 0 et 2.Pi/A avec A pas des récepteurs et avec C variable pour que A.C varie sur [-10% ;+10%].

On obtient alors une Norme fonction de f0,k0 et C, f0 et k0 étant fixés :

$$Norme(f0, k0, C) = \sum_{n=1}^{R} |\langle e_{test}(k0, C)|U_n(f0)\rangle|^2$$

Dont on cherche le maximum pour C=Cmax sur le domaine de C, pour f0 et k0 fixés.

Il suffit alors pour représenter les modes de propagation d'un guide d'épaisseur lentement variable, de façon indépendante de l'angle du dièdre du guide, de reporter sur un graphique en deux dimensions, la valeur du maximum de la norme trouvé Norme(f0,k0,Cmax) qui est entre 0 et 1, pour le point (f0,k0) choisi dans le plan f-k.

Enfin, il suffira de choisir d'autres points (f1,k1), ... (fn,kn) dans le plan f-k pour tracer une représentation des modes de propagation du guide d'épaisseur lentement variable, indépendamment de la variation d'épaisseur du guide.

Il en résulte une représentation robuste des modes de propagation d'un guide d'épaisseur lentement variable.

L'invention a été testée pour des angles de dièdre de 1° et 2° qui sont représentatifs des angles de dièdre d'un os cortical long comme le radius humain.

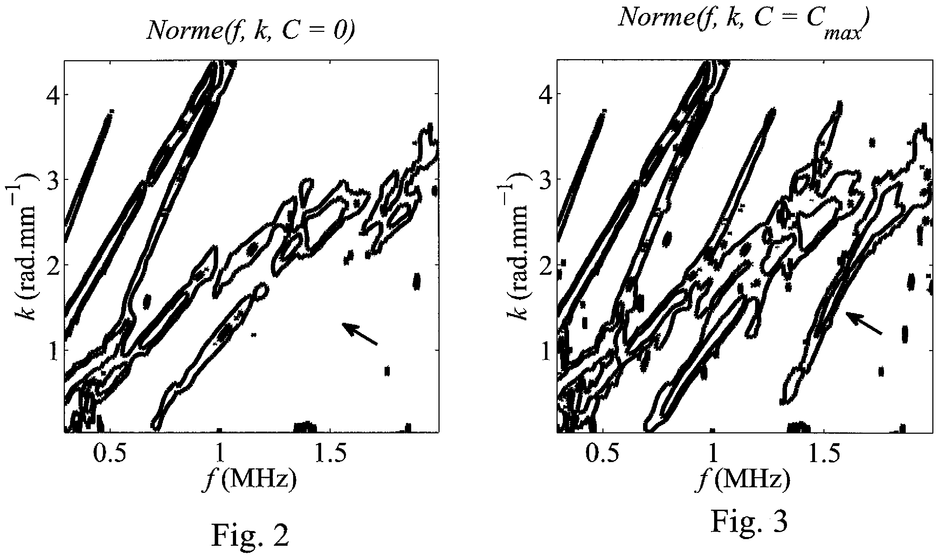

Sur les figures 2 et 3, la représentation choisie pour la propagation guidée, est en lignes de niveau avec un seuil supérieur à 0,5, pour les valeurs retenues pour Norme(f,k,C=0) et Norme(f,k,C=Cmax). En chaque point, si Norme(f,k,C=0) < 0,5, sur la figure 2, ou Norme(f,k,C=Cmax) < 0,5,sur la figure3, la courbe de niveau de Norme(f,k,C=Cmax) n'est ainsi pas représentée. Les courbes de niveau 0,5 et 0,7 sont ainsi représentées sur ces figures.

Sur la figure 2, la représentation des modes de propagation d'un guide avec un angle de dièdre de 2° dans l'art antérieur est présentée par les valeurs de Norme(f,k,C=0). On note une absence de mode de propagation dans une zone définie par f>1,5Mhz et k<2 rad.mm-1.

Sur la figure 3, la représentation des modes de propagation du même guide avec un angle de dièdre de 2° et avec application de l'invention est présentée par comparaison, par les valeurs de Norme(f,k,C=Cmax).

Il est observé que la structure de l'ensemble des modes est conservée globalement, entre les deux figures pour les valeurs faibles de f et k.

Toutefois, au moins un mode, dont la position est indiquée par une flèche sur les figures 2 et 3, et qu'il est possible de retrouver par simulation par éléments finis, comme étant présent dans la zone f>1,5 MHz et k<2rad.mm-1, est nettement révélé sur la figure 3 par l'invention alors qu'il n'est pas observable sur la figure 2. Il est possible de comparer les modes obtenus avec les modes théoriques d'une plaque d'épaisseur constante égale à eO, épaisseur au niveau du milieu du segment des récepteurs.

On peut ainsi vérifier que pour une sonde ultrasonore donnée, par rapport à un cas théorique calculable ou simulable numériquement, les modes de propagation d'un guide en dièdre sont effectivement représentés en plus grand nombre avec l'invention qu'avec l'art antérieur.

L'invention présente donc un avantage dans la représentation des modes de propagation d'un guide d'épaisseur lentement variable, au sens de l'approximation adiabatique et donc pour l'étude de la partie corticale d'un os humain réel.

L'invention est donc susceptible d'application industrielle ou utile dans l'aide à l'évaluation d'un risque de fracture de l'ostéoporose ou d'autres maladies osseuses.

Au sens de la demande, seront considérés comme des équivalents des capteurs ultrasonores piézo-électriques décrits, notamment les capteurs suivants :

- capteurs CMUT (« Capacitive micromachined ultrasonic transducers ») ;

- capteurs utilisant des lasers à la fois pour émettre une onde ultrasonore et la détecter.

Au sens de la demande, seront considérés comme un équivalent à une représentation dans un repère f-k de la propagation des ondes ultrasonores dans un guide, la représentation dans un repère fréquence-vitesse de phase de la propagation de ces ondes.

Au sens de la présente demande, il sera considéré équivalent d'utiliser un nombre d'émetteurs N supérieur au nombre M des récepteurs pour la mise en oeuvre de l'invention, le rang numérique R étant choisi inférieur dans ce cas inférieur ou égal à M. Le rang R sera ainsi choisi, dans tous les cas de l'invention, inférieur ou égal au plus petit du nombre N des émetteurs et du nombre M des récepteurs.

Au sens de la présente demande, il sera considéré équivalent d'échanger le rôle des émetteurs et des récepteurs, pour la mise en oeuvre de l'invention, l'utilisation des vecteurs singuliers de réception étant alors remplacée par l'utilisation des vecteurs singuliers d'émission dans l'exposé ci-dessus.

Au sens de la présente demande, il sera considéré équivalent pour la mise en oeuvre de l'invention, que la barrette soit en contact avec le guide ou soit séparée de celui-ci par des tissus mous, tout en étant parallèle à la surface du guide la plus proche de cette barrette.

Au sens de la présente demande, des « moyens de calcul numériques» pourront notamment être constitués d'un ordinateur exécutant un programme ou comprendre un tel ordinateur.

## Revendications

1. Dispositif pour la représentation, dans un repère fréquence-nombre d'onde f-k, de la propagation d'une onde ultrasonore dans un guide en dièdre (1), **caractérisé en ce qu'**il comprend des émetteurs ultrasonores (2) référencés par « Ej » avec j entier variant entre 1 et N, N entier strictement positif et des récepteurs ultrasonores (3) référencés par « Ri » avec i entier variant entre 1 et M, M entier strictement positif, les récepteurs étant disposés spatialement sur un premier segment d'une droite selon un pas régulier « A », **en ce qu'**il comprend des moyens de traitement du signal reçu par les récepteurs, en provenance des émetteurs, et **en ce que** les moyens de traitement comprennent des moyens de calcul d'une transformée de Fourier spatiale discrète modifiée, pour une variable spatiale d'intégration « x », centrée au milieu dudit premier segment et parcourant les récepteurs dans le sens des x croissants, et pour un vecteur d'onde k(x) égal à un produit k.P(x), avec k coefficient constant en x et compris entre 0 et 2*Pi/A, et avec P(x) polynôme en x, de coefficient de degré 0 en x égal à 1 et de coefficient « C » de degré 1 en x tel que C.A est compris entre -1/10 et +1/10.

2. Dispositif selon la revendication 1 dans lequel les émetteurs sont disposés sur ladite droite.

3. Dispositif selon la revendication 2 dans lequel les émetteurs sont disposés sur ladite droite selon le pas «A» et forment avec les récepteurs une barrette s'étendant linéairement dans la direction de ladite droite.

4. Dispositif selon la revendication 1 dans lequel les émetteurs et les récepteurs sont des capteurs piézo-électriques.

5. Dispositif selon la revendication 1 dans lequel les moyens de traitement du signal comprennent des convertisseurs d'un signal analogique en signal numérique

6. Dispositif selon la revendication 1 dans lequel les moyens de calcul sont numériques

7. Procédé d'utilisation du dispositif selon la revendication 1 pour la représentation, dans un repère fréquence-nombre d'onde f-k, de la propagation d'une onde ultrasonore dans un guide en dièdre, à une fréquence temporelle f0 et à un nombre d'onde k0, comprenant les étapes suivantes :

- émettre dans le guide, un signal ultrasonore fonction du temps et à large bande passante incluant la fréquence « f0 », par un émetteur Ej avec j entier choisi entre 1 et N;
- recevoir, le signal propagé dans le guide, en fonction du temps, sur les récepteurs Ri avec i entier choisi entre 1 et M;
- calculer une transformée de Fourier temporelle discrète du signal reçu à la fréquence f0 et ranger le résultat Sij dans la i-ième ligne et la j-ième colonne d'une matrice rectangulaire S(f0) à M lignes et N colonnes;
- remplir la matrice S(f0), en répétant les opérations précédentes pour les récepteurs autres que Ri et pour les émetteurs autres que Ej;
- décomposer la matrice S(f0) en valeurs singulières en fixant un rang numérique « R » de la matrice S, avec R entier choisi entre 1 et N, pour obtenir un nombre R de vecteurs singuliers de réception référencés par « Ur (f0)» avec r entier variant entre 1 et R ;
- calculer une transformée de Fourier spatiale discrète modifiée des vecteurs singuliers de réception Ur (f0), pour une variable spatiale d'intégration « x » centrée au milieu dudit premier segment et parcourant les récepteurs et pour un vecteur d'onde k(x) égal à k0, choisi entre 0 et 2.Pi/A, multiplié par un polynôme en x « P(x) », de coefficient de degré 0 égal à 1 et de coefficient « C » de degré 1 en x tel que C.A est variable entre -1/10 et +1/10 ;
- calculer une fonction « Norme(f0,k0,C) », égale à la somme des modules carrés des transformées de Fourier spatiales discrètes modifiées des vecteurs singuliers de réception Ur (f0) sur la plage de variation de C,
- déterminer la valeur C=Cmax pour laquelle Norme(f0,k0,C) est maximum, sur la plage de variation de C;
- représenter dans le repère f-k, la propagation de l'onde ultrasonore dans le guide en dièdre, en f0 et k0, en reportant la valeur du maximum Norme(f0,k0,Cmax) au point (f0,k0) du repère.

8. Application du procédé selon la revendication 7 pour obtenir la représentation, dans le repère f-k, de la propagation des ondes ultrasonores dans le guide en dièdre, en reportant la valeur du maximum Norme(f,k,Cmax(f,k)) au point (f,k) du repère, pour k variant entre 0 et 2.Pi/A et f variant sur les fréquences de la large bande passante.

9. Application du procédé selon la revendication 8 dans laquelle le guide en dièdre est un os long humain.

**Patentansprüche**

1. Vorrichtung, um in einem Frequenz-Wellenzahl Referenzsystem f-k die Ausbreitung einer Ultraschallwelle in einem V-förmigen Wellenleiter (1) darzustellen, **dadurch gekennzeichnet, dass** sie Ultraschallsender (2) mit der Bezeichnung "Ej" aufweist, wobei j eine ganze Zahl zwischen 1 und N ist, wobei N strikt positiv ist, und Ultraschallempfänger (3) mit der Bezeichnung "Ri" aufweist, wobei i eine ganze Zahl zwischen 1 und M ist, wobei M strikt positiv ist, wobei die Empfänger räumlich auf einem ersten Abschnitt einer Geraden nach einer regelmäßigen Teilung "A" angeordnet sind, dass sie Mittel für die Verarbeitung des von den Empfängern erhaltenen Signals der Sender umfasst und dass die Verarbeitungsmittel Mittel für die Berechnung einer modifizierten diskreten räumlichen Fouriertransformation für eine räumliche Integrationsvariable "x" umfasst, die in der Mitte des ersten Abschnitts zentriert ist und die Empfänger in der Richtung der ansteigenden x durchläuft, und für einen Wellenvektor k(x), der gleich einem Produkt k.P(x) ist, wobei k ein in x konstanter Koeffizient zwischen 0 und 2*Pi/ A ist und P(x) ein Polynom in x mit einem Koeffizienten mit Grad 0 in x gleich 1 und einem Koeffizienten "C" mit Grad 1 in x, so dass C.A zwischen -1/10 und +1/10 liegt.

2. Vorrichtung nach Anspruch 1, bei der die Sender auf der Geraden angeordnet sind.

3. Vorrichtung nach Anspruch 2, bei der die Sender auf der Geraden nach der Teilung "A" angeordnet sind und mit den Empfängern eine Leiste bilden, die sich linear in Richtung der Gerade erstreckt.

4. Vorrichtung nach Anspruch 1, bei der die Sender und die Empfänger piezoelektrische Sensoren sind.

5. Vorrichtung nach Anspruch 1, bei der die Signalverarbeitungsmittel Analog-Digital-Signalwandler umfassen.

6. Vorrichtung nach Anspruch 1, bei der die Rechenmittel digital sind.

7. Verfahren für die Verwendung der Vorrichtung nach Anspruch 1, um in einem Frequenz-Wellenzahl Referenzsystem f-k die Ausbreitung einer Ultraschallwelle in einem V-förmigen Wellenleiter darzustellen bei einer zeitlichen Frequenz f0 und einer Wellenzahl k0 mit folgenden Schritten:

- Senden eines Ultraschallsignals in den Wellenleiter, das von der Zeit abhängig ist und eine hohe Bandbreite mit der Frequenz "f0" aufweist, durch einen Sender E, wobei j eine zwischen 1 und N gewählte ganze Zahl ist,
- Empfangen des in dem Wellenleiter ausgebreiteten Signals in Abhängigkeit von der Zeit an den Empfängern i, wobei i eine zwischen 1 und M gewählte ganze Zahl ist,
- Berechnen einer modifizierten diskreten räumlichen Fouriertransformation des empfangenen Signals für die Frequenz f0 und Einordnen des Ergebnisses Sij in die ite Zeile und jte Spalte einer rechteckigen Matrix S(f0) mit M Zeilen und N Spalten,
- Füllen der Matrix S(f0) durch Wiederholung der vorigen Schritte für die Empfänger außer Ri und für die Sender außer Ej,
- Zerlegen der Matrix S(f0) in Singulärwerte durch Festlegung eines digitalen Rangs "R" der Matrix S, wobei R eine zwischen 1 und N gewählte ganze Zahl ist, um eine Anzahl R von Empfangssingulärvektoren mit der Bezeichnung "Ur (f0)" zu erhalten, wobei r eine zwischen 1 und R gewählte ganze Zahl ist,
- Berechnen einer modifizierten diskreten räumlichen Fouriertransformation der Empfangssingulärvektoren Ur (f0) für eine räumliche Integrationsvariable "x", die in der Mitte des ersten Abschnitts zentriert ist und die Empfänger durchläuft, und für einen Wellenvektor k(x), der gleich k0 ist, gewählt zwischen 0 und 2*Pi/A, multipliziert mit einem Polynom in x "P(x)" mit einem Koeffizienten mit Grad 0 gleich 1 und einem Koeffizienten "C" mit Grad 1 in x, so dass C.A zwischen -1/10 und +1/10 variiert,
- Berechnen einer Funktion "Norm(f0,k0,C)", die gleich der Summe der Quadratmodule der modifizierten diskreten räumlichen Fouriertransformationen der Empfangssingulärvektoren Ur (f0) über den Variationsbereich von C ist,
- Bestimmung des Werts C=Cmax, bei dem Norm(f0,k0,C) maximal ist, über den Variationsbereich von C,
- Darstellen in dem Referenzsystem f-k der Ausbreitung der Ultraschallwelle in dem V-förmigen Wellenleiter in f0 und k0 durch Übertragen des Werts des Maximums Norm(f0,k0,Cmax) auf den Punkt (f0,k0) des Referenzsystems.

8. Anwendung des Verfahrens nach Anspruch 7, um in dem Referenzsystem f-k die Ausbreitung der Ultraschallwellen in dem V-förmigen Wellenleiter zu erhalten durch Übertragen des Werts des Maximums Norm(f,k,Cmax(f,k)) auf den Punkt (f,k) des Referenzsystems, wobei k zwischen 0 und 2.Pi/A variiert und f über die Frequenzen der hohen Bandbreite variiert.

9. Anwendung des Verfahrens nach Anspruch 8, bei der der V-förmige Wellenleiter ein langer menschlicher Knochen ist.


**Claims**

1. A device for the representation, in a frequency-wavenumber frame f-k, of the propagation of an ultrasound wave in a dihedral guide (1), **characterized in that** it comprises ultrasound emitters (2) referenced by "Ej" with j an integer varying between 1 and N, N a strictly positive integer and ultrasound receivers (3) referenced by "Ri" with i an integer varying between 1 and M, M a strictly positive integer, the receivers being disposed spatially over a first segment of a straight line according to a regular pitch "A", **in that** it comprises means for processing the signal received by the receivers, coming from the emitters, and **in that** the processing means comprise means for calculating a modified discrete spatial Fourier transform, for a spatial integration variable "x", centered in the middle of said first segment and traversing the receivers in the direction of increasing x, and for a wave vector k(x) equal to a product k.P(x), with k a coefficient which is constant in x and between 0 and 2*Pi/A, and with P(x) a polynomial in x, of coefficient of degree 0 in x equal to 1 and of coefficient "C" of degree 1 in x such that C.A is between - 1/10 and + 1/10.

2. The device according to claim 1, wherein the emitters are disposed on said straight line.

3. The device according to claim 2, wherein the emitters are disposed on said straight line according to the pitch "A" and form with the receivers an array extending linearly in the direction of said straight line.

4. The device according to claim 1, wherein the emitters and the receivers are piezoelectric sensors.

5. The device according to claim 1, wherein the signal processing means comprise converters for converting analog signals to digital signals.

6. The device according to claim 1, wherein the calculation means are digital.

7. A method of using the device according to claim 1 for the representation, in a frequency-wavenumber frame f-k, of the propagation of an ultrasound wave in a dihedral guide, at a temporal frequency f0 and at a wavenumber k0, comprising the following steps:

    - emitting in the guide, a time-dependent broadband ultrasound signal including the frequency "f0", by an emitter Ej with j a chosen integer between 1 and N;
    - receiving the signal propagated in the guide, as a function of time, on the receivers Ri with i a chosen integer between 1 and M;
    - calculating a discrete temporal Fourier transform of the signal received at the frequency f0 and placing the result Sij in the i-th row and the j-th column of a rectangular matrix S(f0) with M rows and N columns;
    - filling the matrix S(f0), by repeating the previous operations for the receivers other than Ri and for the emitters other than Ej;
    - decomposing the matrix S(f0) into singular values by fixing a numerical rank "R" of the matrix S, with R a chosen integer between 1 and N, to obtain a number R of singular reception vectors referenced by "Ur (f0)" with r an integer varying between 1 and R;
    - calculating a modified discrete spatial Fourier transform of the singular reception vectors Ur (f0), for a spatial integration variable "x" centered in the middle of said first segment and traversing the receivers and for a wave vector k(x) equal to k0, chosen between 0 and 2.Pi/A, multiplied by a polynomial in x "P(x)", of coefficient of degree 0 equal to 1 and of coefficient "C" of degree 1 in x such that C.A is variable between - 1/10 and + 1/10;
    - calculating a function "Norm(f0,k0,C)", equal to the sum of squared modules of the modified discrete spatial Fourier transforms of the singular reception vectors Ur (f0) over the range of variation of C;
    - determining the value C=Cmax for which Norm(f0,k0,C) is maximum, over the range of variation of C;
    - representing in the frame f-k, the propagation of the ultrasound wave in the dihedral guide, in f0 and k0, by referring the value of the maximum Norm(f0,k0,Cmax) to the point (f0,k0) of the frame.

8. An application of the method according to claim 7 to obtain the representation, in the frame f-k, of the propagation of the ultrasound waves in the dihedral guide, by referring the value of the maximum Norm(f,k,Cmax(f,k)) to the point (f,k) of the frame, for k varying between 0 and 2.Pi/A and f varying over the frequencies of the broadband.

9. An application of the method according to claim 8, wherein the dihedral guide is a human long bone.

Fig. 1

Fig. 3

Fig. 2

## RÉFÉRENCES CITÉES DANS LA DESCRIPTION

**Documents brevets cités dans la description**

- FR 2946753